# EUROPEAN PATENT APPLICATION

(11) **EP 2 111 845 A1**
(43) Date of publication of application: **28.10.2009**
(21) Application number: 09155586.2
(22) Date of filing: 19.03.2009
(51) Int. Cl.: A61K 8/25, A61K 8/34, A61K 8/04, A61Q 17/00

(54) **Sliding preventive compound**

(30) Priority: 25.04.2008 AR P800001017
(71) Applicant: Warcok, Maximiliano, CP 1419 Buenos Aires (AR)
(72) Inventor: Warcok, Maximiliano, CP 1419 Buenos Aires (AR)
(74) Representative: Robba, Pierpaolo

(57) **Abstract**

A sliding preventive compound such as the one caused in human beings during the exudation of liquids through perspiration either in sports activities or in any other activity that generates perspiration, being the compound **characterized in that** it contains from 0.1 to 30% in water weight, from 60 to 99% in ethyl alcohol weight and 0.9 to 20% in silicon oxide weight.

## Description

This invention refers to a sliding preventive compound such as the one caused in human beings during the exudation of liquids through perspiration when performing sports activities or any other activity that causes perspiration.

As it is known, when performing any sports activities, the human body releases water and salts in the form of sweat, which is used by our organism as a vehicle for the elimination of toxins. The sweat or perspiration is accumulated in different parts of the body, such as the armpit, thorax, hands, feet, etc, providing said parts a greater humidity and greasiness sensation, particularly in hands and feet. This particular increase of perspiration involves a substantial decrease of the normal adherence that feet and hands naturally have.

The problem of the lack of adherence caused by perspiration is widely known in the sports world and the industry since it causes a decrease in the physical and/or labor performance, besides accidents, and can also cause problems when performing daily activities.

On the other hand, the problem in the feet due to the excess in perspiration is not minor. The sweat forms over the sole of the foot a fine coat of liquid between the foot and the shoes or socks. The constant movement and the temperature makes the foot to slide in the shoe and cause rubbing or folds in the sock, thus causing discomfort, blisters or injures. This particular problem is well known by rugby, football, tennis players, athletes and sportsmen in general or people who must walk, jog or run for carrying out a specific labor, as for example, post man, collection employees, refuse collectors, etc.

It is important to point out that in the hands the sweat forms a fine liquid coat over the sole of the hands which prevents us to hold objects, loosing the adherence in the same and causing injuries, serious in same cases, due to glove sliding over the hand, force excess, involuntary sliding of the object or due to the simple excess of lubrication and transfer of humidity and greasiness towards the object.

As an example we can mention many accidents related to the excess of perspiration in daily activities. For example in the sliding of gloves, this effect is produced by the accumulation of perspiration between the glove and the hand. The temperature and movement generates a rubbing that cause discomfort and blisters. This problem is well-known by doctors, surgeons, health personnel, laboratory chemistry, motorists, operators, firemen, motorcyclists, boxers, golfers, goalkeepers and sportsmen in general.

In case of excess of force, this is produced to compensate the lack of adherence of the hand with the object in question. Said excess of force is not only produced in the hand, but in the whole body, producing injures due to the fact that different positions are adopted to avoid the falling of the object or of the person in case said object is the point of support. This is known by body builders, weight lifters, climbers, lancers or any other person who needs to lift or hold a heavy object.

The problems due to involuntary sliding are also known. Particularly in the case in which objects with little rough surfaces are manipulated (for example, plated materials, steel sheets, glass materials, acrylic surfaces, etc. This is known by operators, moving employees, mechanicals and cleaning personnel.

As an additional example, we can mention the accidents due to an excess of lubrication and perspiration transfer and other sources of grease or humidity to the object, particularly, when the object must pass from hand to hand. This is well known by the operators in production line, mechanics and any other activity in which objects are transferred from hand to hand and where the same gets dirty. For example, remote controls, joysticks, working tools, etc.

A partial solution to the problems mentioned above is given by products for cleaning and/or drying which are known.

In case of increasing the adherence for hands, there are many products in the market, as for example resins, powders, talcum powder and other agents for cleaning and/or drying, and antiperspirant for hands.

In the case of resins, which are sticky, they have a short duration and sometimes are difficult to remove from the skin. Powder and talcum powder act only in absorbing the humidity from hands and feet, but they do not remove the grease, they do not leave an anti-sliding coat nor have disinfectant activity. Further, under some circumstances of humidity, these products become doughy, lumpy and difficult to apply. Antiperspirants for hands are made of aluminum salts, with botox or other techniques that prevent sweat release, with its toxins, outside the body either for blocking the pores or other action, which is not advisable for the correct removal of toxins. Cleaning and drying agents remove the superficial fat and humidity but do not form an anti-sliding film.

Thus, it is an object of this invention to provide sliding preventive compound such as the caused by human beings during the exudation of liquids through perspiration either in sports activities or in any other activity that generates perspiration.

Thus, it is an object of this invention to provide sliding preventive compound including the application properties described above in this specification, since this problem can only be solved by means of methods such as constant cleaning, washing of hands and feet, dressing, etc. It is evidently advantageous to try to resolve a problem such as the one described above, so that the product can efficiently act in the area of origin of said problem without being detrimental to the desired conditions for different parts of the human body.

It is further an object of this invention to provide a sliding preventive compound such as the caused by human beings during the exudation of liquids through perspiration either in sports activities or in any other activity that generates perspiration, and further being a grease remover, antiseptic and non-sticky product which does not block the pores, as well as a product that generates few residue, does not stain and easy to remove.

It is also an object of this invention to provide sliding preventive compound such as the one caused in human beings during the exudation of liquids through perspiration either in sports activities or in any other activity that generates perspiration, notably reducing the possibility of formation of blister for rubbing, injures and simple discomfort in feet and hands.

The compound of this invention has a formulation that contains from 0.1 to 30% in water weight, 60 to 99% in ethyl alcohol weight and from 0.9 to 20% in pirogenic silicon oxide, such as aerosil®, cabosil® or the like. It is important to point out that the percentages mentioned above can be modified by adding other ingredients, such as perfumes, antibacterial agents, etc., which modify the proportions pursuant to the application field.

Once the compound of this invention has been placed on the skin, it immediately gives an adherence sensation to the shoes or socks and great comfort. It is important to point out that any person with knowledge in this field will appreciate that the compound of this invention can be commercialized and distributed in a wide range of presentations, such as solutions, aerosol or gel packaged in bottles with which the necessary quantities are easily dosed to be applied in the desired zones of the body. After its application, a few seconds must pass so that the alcohol dries and a non-sliding film is formed and thus the desired effect is achieved. It is important to mention that the compound is dermatological acceptable. Its effectiveness must have an adequate duration and an easy and comfortable application.

## Claims

1. A sliding preventive compound such as the one caused in human beings during the exudation of liquids through perspiration either in sports activities or in any other activity that generates perspiration, being the compound **characterized in that** it contains from 0.1 to 30% in water weight, from 60 to 99% in ethyl alcohol weight and from 0.9 to 20% in silicon oxide.

2. The compound according to claim 1, **characterized in that** it further contains gelling agents from 0.1 to 5% in weight as polysaccharides, carboximethylcellulose, hidroximethylcellulose.

3. The compound according to claim 1, **characterized in that** it further contains essences and colorants in a concentration from 0.01 to 5% in weight.

4. The compound according to claim 1, **characterized in that** it has anti-sliding characteristics.

5. The compound according to claim 1, **characterized in that** it is in semisolid state.

6. The compound according to claim 1, **characterized in that** it is in liquid state.

7. The compound according to claim 5, **characterized in that** said semisolid state contains from 5 to 10% in water weight, from 80 to 66% in ethyl alcohol weight and from 5 to 20% in silicon oxide weight.

8. The compound according to claim 6, **characterized in that** said liquid state contains from 10 to 20% in water weight, from 60 to 75% in ethyl alcohol weight and from 1 to 5% in silicon oxide weight.
